Europäisches Patentamt

European Patent Office

Office européen des brevets

(11) Veröffentlichungsnummer: **0 008 036**
A1

# EUROPÄISCHE PATENTANMELDUNG

(21) Anmeldenummer: 79102619.8

(51) Int. Cl.³: **A 21 D 2/36**

(22) Anmeldetag: 24.07.79

(30) Priorität: 26.07.78 DE 2832782
25.01.79 DE 2902896

(71) Anmelder: Kleinert, Geerd, Malzbendener Aul 1, D-5372 Schleiden-Gemünd (DE)

(43) Veröffentlichungstag der Anmeldung: 20.02.80
Patentblatt 80/4

(72) Erfinder: Kleinert, Geerd, Malzbendener Aul 1, D-5372 Schleiden-Gemünd (DE)
Erfinder: Kleinert, Armin, Franz Elfeler Weg 8, D-5310 Gellenkirchen (DE)

(84) Benannte Vertragsstaaten: AT BE CH FR IT LU NL SE

(74) Vertreter: Schrumpf, Frithjof, Dipl.-Chem., Koenenstrasse 20 Postfach 731, D-5160 Düren (DE)

(54) Verfahren zur Herstellung von Kräuterbrot.

(57) Ein Kräuterbrot wird hergestellt, indem man getrocknete Kräuter in Form eines trockenen Pulvers mit einem Zerkleinerungsgrad entsprechend DAB 6 Sieb Nr. 6 in einer Menge bis zu 6 Gew.%, vorzugsweise 1,5 Gew.%, der trockenen Mehl- bzw. Backmischung zusetzt und diese in an sich bekannter Weise fertigstellt und ausbäckt.

Vorzugsweise wird das Brot biologisch, insbesondere durch Hefe gelockert.

Es zeichnet sich durch gute organoleptische Eigenschaften, lockere, elastische Krume und hohe Haltbarkeit aus. Ferner kann es als galenische Zubereitung eingesetzt werden.

EP 0 008 036 A1

0008036

- 1 -

23.7.1979
K 267

Geerd Kleinert
5372 Schleiden-Gemünd


Verfahren zur Herstellung von Kräuterbrot


Die Erfindung betrifft ein Verfahren zur Herstellung von Kräuterbrot.

Es ist bekannt, den Teig von bestimmten Backwaren, wie Kümmelbrot oder Anisplätzchen, mit zerkleinerten Gewürz-Drogen zu versetzen. Diese Maßnahme dient jedoch lediglich zur Erzielung von Geschmacks- und/oder Geruchs-nuancen; irgendwelche backtechnischen oder pharmakolo-gischen Effekte wurden weder angestrebt noch beobachtet.

Insbesondere ist das Einverleiben von Heilkräutern in Backwaren bei Arzneidrogenpräparaten als Zubereitungsform unbekannt und wird von den Pharmakopöen auch nicht vorgesehen.

Im Gegenteil sollen nach herrschender Meinung Galenika die Wirkstoffe in sogenannter therapeutisch wertvoller Form, möglichst befreit von Ballaststoffen, enthalten. Als feste Zubereitungen sind allenfalls Frischpflanzenverreibungen mit Zucker und anderen Trägersubstanzen vorgesehen. Es fehlt jedoch an einem festen Träger, der nicht nur eine zuverlässige Dosierung ermöglicht, sondern auch die perorale Verabreichung erleichtet und vereinfacht.

Es wurde nun überraschenderweise gefunden, daß der Zusatz von Kräuterpulver in der bestimmten Feinheit und Konzentration bei bakteriologischer Lockerung des Teiges zu einer wesentlichen Qualitätsverbesserung des erhaltenen Brotes führt. Insbesondere lässt sich die Wasseraufnahme des Teiges erheblich steigern, und außerdem wird ein besserer Trieb erzielt, verglichen mit einem Teig ohne Kräuterpulverzusatz. Die Elastizität des Brotes ist ebenfalls sehr viel höher. Ein weiterer, unerwarteter Effekt des erfindungsgemäßen Kräuterpulver-Zusatzes ist, daß sich die Porosität des Brotes von fein bis grob einstellen lässt, in jedem Fall aber eine gute Gleichmäßigkeit aufweist.

Ferner wurde überraschenderweise gefunden, daß der normale Backvorgang die Wirkstoffe von Arzneimitteldrogen nicht oder kaum mehr beeinträchtigt als Abkochungen oder andere Zubereitungsformen. Auch die anderen Forderungen von J. Büchi (Arch. Pharm. 285,40, 1952) werden weitgehend erfüllt. Bei Dauerbackwaren ist insbesondere eine gute Haltbarkeit gegeben. Im Gegenteil scheint in einigen Fällen sogar eine Aktivierung der Wirkstoffe einzutreten, die möglicherweise auf biologische oder chemische Reaktion während der Teiglockerung zurückzuführen ist.

Praktische Untersuchungen haben darüberhinaus gezeigt, daß sich die Wirkstoffe der Heilkräuter bei den angegebenen Zerkleinerungsgrad des Pulvers während der Gärung und dem Backvorgang auch ohne vorherige Extraktion voll entfalten können und daß insbesondere die eingebrachten ätherischen Öle weitgehend erhalten bleiben.

Die Erfindung hat sich die Aufgabe gestellt, ein Verfahren zur Herstellung von Kräuterbrot zu schaffen, das backtechnisch einfach durchführbar ist und gleichwohl eine gute Verwertung der Kräuterbestandteile sicherstellt.

Diese Aufgabe wird erfindungsgemäß dadurch gelöst, daß man getrocknete Kräuter in Form eines trockenen Pulvers mit einem Zerkleinerungsgrad entsprechend DAB 6 Siebnummer 6 in einer Menge bis zu 6 Gew.%, vorzugsweise 1,5 Gew.% der trockenen Mehl- bzw. Backmischung zusetzt und diese in an sich bekannter Weise fertigstellt und ausbäckt.

Die Siebnummer 6 ist für zerkleinerte Drogen durch das Deutsche Arzneibuch Nr. 6 definiert und entspricht annähernd 0,15 mm Maschenweite.

Der hier und im folgenden verwendete Ausdruck "Kräuterbrot" soll allgemein für alle Arten von Backwaren gelten, wenn auch Hefe- und Sauerteig-Gebäcke bevorzugt werden.

Weitere Ziele, Merkmale und Vorteile der Erfindung ergeben sich aus der nachfolgenden Beschreibung und den Unteransprüchen.

Die Erfindung wird durch das nachfolgende Beispiel erläutert:

Die folgende Kräutermischung wird zusammengestellt (Angaben in Gewichtsprozent)

| | |
|---|---|
| Schlehdorn | 10 |
| Holunder | 6 |
| Bitterklee | 6 |
| Sennesblätter | 30 |
| Sennesschoten | 25 |
| Fenchel | 4 |
| Kümmel | 3 |
| Ginster | 10 |
| Stiefmütterchen | 6 |

Diese Drogen werden schonend getrocknet und pulverisiert. Die ein Sieb Nr. 6 gemäß DAB 6 passierende Fraktion wird zu einem Backversuch weiter verwendet. Hierzu werden 15 g des Kräuterpulvers gleichmäßig in 1000 g Roggenmehl eingemischt. Unter Zusatz von 50 g Hefe und 850 g Wasser sowie etwas Salz werden nach an sich bekannter Teigführung und Weiterbehandlung drei Roggenbrote ausgebacken. Das Brot ist im Geschmack angenehm und hat eine verdauungsregelnde Wirkung, die harmonischer ist als die eines Aufgusses aus den genannten Bestandteilen. Die Brotkrume fördert die Peristaltik; tiefgekühlt ist das Brot lange Zeit haltbar. Bei normaler Lagerung bleibt es etwa doppelt solange frisch wie ein Brot ohne Kräuterpulverzusatz. Überdies gestattete das verwendete Mehl unter sonst gleichen Ansatz- und Temperaturbedingungen maximal die Zugabe von etwa 660 g Wasser.

Mit Kräuter-Preßsaft oder -Extrakt ließen sich die backtechnischen Vorteile des erfindungsgemäßen Verfahrens nicht erzielen; auch sind die damit erzeugten Backwaren organoleptisch unbefriedigend, und sie zeigen - wenn die gleiche Kräutermenge zu Grunde gelegt wird - eine geringere pharmakologische Wirkung als die Produkte des erfindungsgemäßen Verfahrens.

Es versteht sich, daß an Stelle oben genannter Kräuter auch andere Gewürz- und/oder Heildrogen verwendet werden können. So sind insbesondere solche Drogen geeignet, die eine Gewichtsreduktion bewirken; in Verbindung mit seinem verminderten Kaloriengehalt eignet sich das erfindungsgemäß hergestellte Kräuterbrot dank seiner gesundheitsfördernden Wirkung und Schmackhaftigkeit gut für diätetische Zwecke.

0008036

23.7.1979
K 267

Geerd Kleinert
5372 Schleiden-Gemünd

Verfahren zur Herstellung von Kräuterbrot

Patentansprüche

1. Verfahren zur Herstellung von Kräuterbrot, dadurch gekennzeichnet, daß man getrocknete Kräuter in Form eines trockenen Pulvers mit einem Zerkleinerungsgrad entsprechend DAB 6 Sieb Nr. 6 in einer Menge bis zu 6 Gew.%, vorzugsweise 1,5 Gew.%, der trockenen Mehl- bzw. Backmischung zusetzt und diese in an sich bekannter Weise fertigstellt und ausbäckt.

2. Verfahren nach Anspruch 1; dadurch gekennzeichnet, daß das Brot biologisch gelockert wird.

3. Verfahren nach Anspruch 2, dadurch gekennzeichnet, daß das Brot durch einen Gärvorgang gelockert wird.

4. Verfahren nach Anspruch 2 oder 3, dadurch gekennzeichnet, daß das Brot mit Hefe gelockert wird.

5. Verfahren nach einem der Ansprüche 1 bis 4, dadurch gekennzeichnet, daß man die Mehl- bzw. Backmischung mit mehr als der bei einem Brot ohne Kräuterpulverzusatz üblichen Wassermenge zu einem Teig verarbeitet.

6. Verfahren nach Anspruch 5, dadurch gekennzeichnet, daß der Wasserüberschuß 1/3 bis 1/2, vorzugsweise etwa 2/5, der normalen Menge beträgt.

7. Verfahren nach einem der vorhergehenden Ansprüche, dadurch gekennzeichnet, daß als Kräuter Heildrogen verwendet werden.

## Europäisches Patentamt

## EUROPÄISCHER RECHERCHENBERICHT

0008036

Nummer der Anmeldung

EP 79 10 2619

| Kategorie | Kennzeichnung des Dokuments mit Angabe, soweit erforderlich, der maßgeblichen Teile | betrifft Anspruch | KLASSIFIKATION DER ANMELDUNG (Int. Cl. 3) |
|---|---|---|---|
| | **EINSCHLÄGIGE DOKUMENTE** | | |
| X | FR - A - 2 269 866 (BUREAU INTER-NATIONAL DE CONSEIL, D'ETUDE ET DE GESTION S.A.R.L.) <br> * Ansprüche 1,5,6,7,8; Seite 2, Zeilen 1-27 * | 1-5,7 | A 21 D 2/36 |
| | -- | | |
| | FR - A - 1 565 295 (ALBERT ALPIN) <br> * Zusammenfassung 1,2,4; Seite 3, Zeilen 1-6 * | 1-4,7 | |
| | -- | | |
| A | DE - A - 2 731 025 (ABERHAM, H.) <br> * Ansprüche 1,3,4 * | 1 | **RECHERCHIERTE SACHGEBIETE (Int. Cl. 3)** <br><br> A 21 D 2/36 |
| | -- | | |
| | DE - C - 881 784 (K. POPP) <br> * Anspruch 1; Seite 2, Zeilen 17-25; Beispiele * | 1-4,7 | |
| | -- | | |
| A | DE - C - 664 393 (C. BRANCO) <br> * Anspruch; Seite 1, Zeilen 12-38 * | 1-4 | |
| | -- | | |
| X | DE - A - 2 609 085 (ULLRICH, H.) <br> * Ansprüche 1-6,9-11; Seite 6, Zeilen 7-8; Seite 7, Zeilen 7-19 * | 1-4,7 | **KATEGORIE DER GENANNTEN DOKUMENTE** <br><br> X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze <br> E: kollidierende Anmeldung <br> D: in der Anmeldung angeführtes Dokument <br> L: aus andern Gründen angeführtes Dokument |
| | ---- | | |
| | Der vorliegende Recherchenbericht wurde für alle Patentansprüche erstellt. | | &: Mitglied der gleichen Patent-familie, übereinstimmendes Dokument |

| Recherchenort | Abschlußdatum der Recherche | Prüfer |
|---|---|---|
| Den Haag | 02-11-1979 | COUCKE |

EPA form 1503.1 06.78